(19) Europäisches Patentamt
European Patent Office
Office européen des brevets

(11) Numéro de publication: **0 091 851**
**B1**

(12) **FASCICULE DE BREVET EUROPEEN**

(45) Date de publication du fascicule du brevet: **19.02.86**

(51) Int. Cl.[4]: **C 07 C 79/355,** C 07 C 103/76, C 07 C 121/75, C 07 C 147/06, C 07 B 37/06

(21) Numéro de dépôt: **83400636.3**

(22) Date de dépôt: **28.03.83**

(54) **Procédé de préparation d'éthers d'aryle portant des substituants différents sur les deux noyaux aromatiques.**

(30) Priorité: **09.04.82 FR 8206379**

(43) Date de publication de la demande: **19.10.83 Bulletin 83/42**

(45) Mention de la délivrance du brevet: **19.02.86 Bulletin 86/8**

(84) Etats contractants désignés: **BE DE GB IT NL**

(56) Documents cités: **FR - A - 1 561 896**

(73) Titulaire: **INSTITUT FRANCAIS DU PETROLE, 4, Avenue de Bois-Préau, F-92502 Rueil-Malmaison (FR)**
Titulaire: **CENTRE NATIONAL DE LA RECHERCHE SCIENTIFIQUE (C.N.R.S.), 15, Quai Anatole France, F-75700 Paris (FR)**

(72) Inventeur: **Jost, Philippe, 11, rue des Volontaires Millery, F-69390 Vernaison (FR)**
Inventeur: **le Perchec, Pierre, 99, rue Pierre Corneille, F-69003 Lyon (FR)**
Inventeur: **Sillion, Bernard, 13, rue des Erables, F-78150 Rocquencourt (FR)**

(74) Mandataire: **Colas des Francs, Jean et al, Institut Français du Pétrole 4, Avenue de Bois Préau, F-92502 Rueil-Malmaison (FR)**

**EP 0 091 851 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L' invention concerne un procédé de préparation d'éthers d'aryle portant des substituants différents sur les deux noyaux aromatiques.

Les dérivés du diphényléther, lorsqu'ils sont substitués par des groupements fonctionnels, sont des produits industriels qui intéressent notamment l'industrie des matières plastiques; en particulier, ils interviennent dans la fabrication de matériaux thermostables. D'autres composés fonctionnalisés sont des intermédiaires d'insecticides à très hautes performances.

Les procédés conduisant à la création de la liaison éther entre deux noyaux aromatiques ne cessent de faire l'objet de travaux en vue d'en améliorer le rendement et/ou la sélectivité.

Il est connu par les travaux d'Ullman qu'un phénate alcalin réagit sur un dérivé halogéné aromatique pour conduire à la formation de l'enchainement éther. Lorsque les noyaux aromatiques ne sont pas substitués, ou sont substitués par des groupements qui ne sont pas attracteurs, la réaction nécessite des conditions sévères. Lorsque la réaction est effectuée avec des dérivés halogénés et des phénates aromatiques portant des groupes attracteurs, la réaction est fortement exothermique et difficile à contrôler, surtout lorsqu'on opère sur des quantités importantes. Dans tous les cas, ces réactions impliquent l'utilisation de deux types de composés différents, le phénate et le dérivé halogéné.

Les brevets allemands 1 290 148 et 2 104 201 décrivent une nouvelle réaction pour la production de diphényléthers substitués par des groupements attracteurs; les résultats de ces travaux sont repris dans une publication de H. Witt, G. Holtschmidt et E. Muller (Angew. Chem. Internat. Edit. 9, 1970, (1) p. 67). Le procédé est basé sur la décomposition thermique baso-catalysée des carbonates de phényle correspondants. L'avantage de ce procédé réside dans le fait que le produit de départ est un produit unique, le phénol substitué, et l'avancement de la réaction, qui se produit en masse, peut être suivi par le dégagement de gaz carbonique.

Ce procédé est particulièrement adapté à la synthèse de diphényléthers substitués sur chaque noyau phényle par des groupes attracteurs identiques tels que CN, $NO_2$, ester carboxylique ou phénylsulfone, mais il ne résout pas le problème de la synthèse des dérivés du diphényléther substitués par deux groupements attracteurs différents; or ces produits peuvent constituer des matières premières importantes pour la préparation de matières plastiques.

L' objet de la présente invention est de fournir un procédé de préparation d'éthers d'aryle de formule générale X-Ar-O-Ar' -Y (I), substitués sur chaque noyau aromatique par un substituant attracteur différent, par une réaction se produisant avec un rendement élevé et une bonne sélectivité. (Ar, Ar', X et Y seront definis dans la description de l'invention qui suit). Le procédé de l'invention consiste d'une manière générale à chauffer ensemble, en une masse fondue ou en solution, des quantités sensiblement équimoléculaires de deux carbonates d'aryle, chacun di-substitué de façon symétrique, et répondant respectivement aux formules générales:

X-Ar-O-CO-O-Ar-X (II)
et Y-Ar' -O-CO-O-Ar' -Y (III)

en présence d'un catalyseur basique.

Alors qu'on aurait pu s'attendre soit à la formation d'un mélange équimoléculaire des deux éthers correspondants X-Ar-O-Ar-X (IV) et YAr' -O-Ar' -Y (V), résultant d'un processus d'élimination intramoléculaire de $CO_2$, soit à la formation d'un mélange des trois éthers (I), (IV) et (V) en proportions statistiques, on observe en fait la formation du produit (I) dissymétrique avec un rendement élevé par rapport aux carbonates de départ.

Cette sélectivité imprévisible, provoquée par un mécanisme qui n'est pas encore élucidé, est à la base de l'invention. Ainsi, l'invention propose un procédé pour la préparation, avec des rendements élevés, d'éthers d'aryle dissymétriques répondant à la formule générale X-Ar-O-Ar' -Y dans laquelle Ar et Ar' représentent chacun un radical aromatique divalent comprenant un ou plusieurs cycles (dérivé par exemple du benzène, du naphtalène ou de l'anthracène — il s'agira de préférence d'un radical phénylène), X et Y représentent chacun un atome ou un groupement électro-attracteur et différent entre eux par leur nature et/ou leurs positions respectives sur les radicaux Ar et Ar', l'un au moins, et de préférence les deux substituants X et Y étant placés sur les radicaux Ar et Ar' de manière à exercer une influence sur la liaison avec l'atome d'oxygène, c'est-à-dire, en position 2 (ortho) ou 4 (para), ou encore en position péri dans le cas où le radical considéré (Ar ou Ar') renferme au moins deux cycles accolés. En outre, il est souhaitable que les groupements électro-attracteurs mis en jeu ne soient pas sensibles à l'action des bases.

Les substituants attracteurs X et Y sont choisis plus particulièrement parmi les groupements $-NO_2$, -CN, -COOAr'', et $-SO_2Ar''$ (où Ar'' représente un radical aromatique monovalent). De préférence, l'un des substituants, par exemple X, est un groupement $-NO_2$, l'autre, Y, pouvant être choisi parmi les groupements mentionnés ci-dessus.

Le procédé de l'invention comprend le chauffage, par exemple à une température de 190 à 280°C, de quantités sensiblement équimoléculaires de deux carbonates d'aryle substitués chacun symétriquement et répondant aux formules générales

X-Ar-O-CO-O-Ar-X (II)
et Y-Ar' -O-CO-O-Ar' -Y (III)

où Ar, Ar', X et Y sont définis comme précédemment, en présence d'un catalyseur basique consistant particulièrement en un sel de métal alcalin, par exemple un carbonate, un phénate, un acétate ou un alcoolate. On utilise le plus souvent le carbonate de potassium. Ce catalyseur est mis en jeu par exemple à raison de 0,1 à 5 moles pour 100 moles de carbonates de départ.

La réaction peut avoir lieu entre les deux carbonates d'aryle à l'état fondu (en masse) ou en solution dans un solvant organique, comme par exemple le nitrotoluène ou l'acétonitrile.

Les carbonates d'aryle substitués symétriquement peuvent être obtenus à partir des phénols correspondants par toute méthode connue.

Parmi les carbonates d'aryle substitués symétriquement que l'on peut faire réagir entre eux dans le procédé de l'invention, on peut mentionner spécifiquement:

— le bis (4-nitrophényl) carbonate;
— le bis (3-nitrophényl) carbonate;
— le bis (4-cyanophényl) carbonate;
— le bis (2-phénoxy-carboxyphényl) carbonate; et
— le bis (4-phénylsulfonyl-phényl) carbonate.

De manière préférée, l'un des deux carbonates d'aryle mis en jeu, par exemple X-Ar-O-CO-O-Ar-X, est substitué symétriquement par deux groupements nitro en position 2 ou en position 4. On utilise par exemple le bis (4-nitrophényl) carbonate. Le second carbonate Y-Ar' -O-CO-O-Ar' -Y peut être alors, de façon spécifique, le bis (3-nitrophényl) carbonate, le bis (4-cyanophényl) carbonate, le bis (2-phénoxy-carboxyphényl) carbonate ou le bis (4-phénylsulfonylphényl) carbonate, déjà mentionnés plus haut. La réaction de décarboxylation, catalysée de préférence par le carbonate de potassium, présente de bonnes sélectivités et des rendements élevés, par exemple d'au moins environ 75% par rapport aux carbonates de départ.

Les éthers d'aryle dissymétriques, obtenus avec de bons rendements par le procédé de l'invention, sont des produits industriels utilisables notamment dans l'industrie des matières plastiques, en particulier dans la fabrication de certains matériaux thermostables.

En particulier, les éthers d'aryle substitués par un ou plusieurs groupements nitro ($-NO_2$) peuvent être soumis à un traitement d'hydrogénation, par exemple en présence de palladium sur charbon, de manière à transformer le(s) groupement(s) nitro en fonction(s) amine(s) primaire(s).

Parmi les produits ainsi obtenus, ceux qui portent soit deux fonctions amines primaires, soit une fonction amine primaire et un groupement carboxylique, sont intéressants dans la préparation de polymères thermostables, par polycondensation sur eux-mêmes (c'est le cas par exemple de l'autocondensation du 2-(4' -aminophénoxy) benzoate de phényle) ou avec des réactifs antogonistes appropriés portant des fonctions amines primaires, des fonctions hydroxyles et/ou des fonctions monocarboxyliques (acides ou esters) ou dicarboxyliques (anhydrides, hémiesters ou diesters vicinaux). A partir de ces divers réactifs, on peut préparer des polycondensats contenant des enchaînements esters ou amides ou encore des enchaînements hétérocycliques, notamment des cycles imides ou quinazolones.

Les exemples suivants illustrent l'invention sans la limiter. Les exemples 2, 5 et 7 sont donnés à titre de comparaison.

*Exemple 1*

*Préparation du 2(4' -nitrophénoxy)benzoate de phényle*

Un mélange de bis(2-phénoxy-carboxy phényl)-carbonate (9,1 g soit 0,02 mole) et de bis(4-nitrophényl)carbonate (6,1 g soit 0,02 mole) est chauffé à 240°C en présence de 0,1 g (0,0007 mole) de $K_2CO_3$. La réaction est très rapide et on recueille 960 cm$^3$ (0,04 mole) de $CO_2$ à 20°C, ce qui est la quantité théorique, en moins de 15 mn. On laisse refroidir le mélange. 80 cm$^3$ d'un mélange (50/50 V/V) toluène-éthanol sont versés et portés à reflux. Après filtration à chaud et refroidissement, un produit cristallise. Il est filtré et séché sous vide. On obtient ainsi 10,3 g d'un produit qui est le 2-(4' -nitrophénoxy)-benzoate de phényle (rendement: 77%, pf: 139°C). En évaporant la solution-mère et en recristallisant l'huile dans 40 cm$^3$ d'éthanol, on obtient un deuxième jet de 1,6 g, ce qui porte le rendement total à 89% de 2-(4' -nitrophénoxy)benzoate de phényle.

*Exemple 2* (comparatif)

*Préparation du 2(4' -nitrophénoxy)benzoate de méthyle*

Un mélange de bis(2-méthoxy-carboxy phényl) carbonate (6,6 g soit 0,02 mole) et de bis(4-nitrophényl)carbonate (6,1 g soit 0,02 mole) est chauffé à 240°C en présence de 0,2 g de carbonate de potassium anhydre (0,0014 mole). La réaction est rapide et, après 20 mn, on laisse refroidir et on introduit 60 cm$^3$ de méthanol, qui est filtré à chaud. Le méthanol est évaporé et une chromatographie colonne préparative du brut réactionnel fournit 36% (3,50 g) de 2-(4' -nitrophénoxy)benzoate de méthyle; pf: 74°C (litt: 76°C). Le rendement est moins bon avec des groupements esters de méthyle portés par l'un des carbonates, qu'avec des groupements esters de phényle, toutes choses égales par ailleurs.

*Exemple 3*

*Préparation du 3,4' di(nitrophényl)éther*

Un mélange de bis(3-nitrophényl)carbonate (6,1 g soit 0,02 mole), de bis(4-nitrophényl)carbonate (6,1 g soit 0,02 mole), et de 0,2 g de carbonate de potassium (0,0014 mole) est porté à 240°C sous agitation à pression ordinaire. On récupère 940 cm$^3$ de $CO_2$ (0,039 mole) en 45 mn. Le mélange est repris avec 120 cm$^3$ d'un mélange éthanol/chloroforme (80/20 volume). On laisse recristalliser, on filtre et on sèche. 7,9 g de 3,4' di(nitrophényl)éther (75%) sont ainsi récupérés. Ce produit analysé en chromatographie liquide haute performance se révèle exempt de 4,4' di(nitrophényl)éther. Il fond à 122°C (litt: 124°C).

Dans cet exemple, les substituants étaient de même nature sur les deux carbonates, mais en position différentes, en méta de la liaison avec l'atome d'oxygène sur l'un, et en para sur l'autre.

*Exemple 4*

*Préparation de 4-(4' -nitrophénoxy)benzonitrile*

Un mélange de bis(4-cyanophényl)carbonate (2,6 g soit 0,01 mole) avec du bis (4-nitrophényl)carbonate (3,0 g soit 0,01 mole) et du carbonate de potassium (0,1 g soit 0,0007 mole) est chauffé à 230°C pendant 1 heure. Le mélange est dissout dans 25 cm$^3$ de toluène, filtré à chaud et cristallisé. On obtient un premier jet de 3,3 g de 4-(4'-nitrophénoxy)benzonitrile (rendement: 69%). En concentrant la solution restante, 0,6 g supplémentaire sont obtenus, ce qui porte le rendement total à 82%. Le produit fond à 150°C.

*Exemple 5* (comparatif)

*Préparation de 4(4' -nitrophénoxy)chlorobenzène*

Un mélange de bis(4-chlorophényl)carbonate (5,7 g soit 0,02 mole), de bis(4-nitrophényl)carbonate (6,1 g soit 0,02 mole) et $K_2CO_3$ (0,2 g soit 0,0014 mole) sont portés à 240°C pendant 4 heures. 400 $cm^3$ de $CO_2$ (soit environ 0,017 mole) sont récupérés. A la fin, on refroidit et on introduit 10 $cm^3$ d'une solution concentrée d'ammoniac et 40 $cm^3$ de dichlorométhane. Après 10 mn, on rajoute à froid 20 $cm^3$ de soude à 5%. La phase organique est décantée, séchée et évaporée. Une chromatographie liquide sur colonne permet de séparer le dinitro-diphényléther du 4(4'-nitrophénoxy)chlorobenzène (pf: 76°C; 1,0 g soit 10% de rendement). Le substituant chlore (non attracteur) ne donne pas lieu à de bons rendements.

*Exemple 6*

*Préparation de 4(4'-nitrophénoxy)diphénylsulfone*

Un mélange de bis(4-phénylsulfonyl phényl)carbonate (4,9 g soit 0,01 mole) avec du bis(4-nitrophényl)carbonate (3,0 g soit 0,01 mole) et du carbonate de potassium (0,1 g soit 0,0007 mole) sont chauffés à 240°C. Après 1 heure, on ajoute 20 $cm^3$ d'acétate d'éthyle qui reflue. On filtre à chaud. Un solide cristallise, il est filtré. On obtient 6,4 g de 4-(4' -nitrophénoxy)diphénylsulfone, qui fond à 126-127°C (rendement: 90%).

*Exemple 7* (comparatif)

*Préparation de 4-(4' -nitrophénoxy)orthophtalate de méthyle*

Un mélange de bis(3,4-di-méthoxycarbonylphényl)carbonate (6,9 g soit 0,0155 mole) avec un bis-(4-nitrophényl)carbonate (4,7 g soit 0,0155 mole) et avec du carbonate de potassium (0,1 g soit 0,0007 mole) est chauffé à 240°C pendant 20 mn, au bout desquelles la quantité théorique de $CO_2$ attendue est atteinte (750 $cm^3$ soit 0,032 mole). Le brut réactionnel est dissout dans 50 $cm^3$ de dichlorométhane et une chromatographie préparative sur silice permet d'obtenir 1,7 g de nitroanisole (0,011 mole), 0,8 g de bis (4-nitrophényl)éther (0,003 mole) et 2,9 g d'un mélange environ 50/50 de 4-(4' -nitrophénoxy)orthophtalate de méthyle et de 4,4' oxy di-orthophtalate de méthyle. Le rendement en éther dissymétrique n'est que de 14%.

*Exemple 8*

*Préparation du 2-(4' -nitrophénoxy) benzoate de phényle*

On opère dans les conditions décrites dans l'exemple 1, mais en utilisant à la place du carbonate de potassium, 0,2 g d'acétate de potassium (soit 0,002 mole).

La réaction conduite à 240°C est achevée en 20 minutes. Le produit est recueilli comme décrit dans l'exemple 1. L' on recueille 9,5 g de 2-(4'-nitrophénoxy) benzoate de phényle (rendement: 71%).

Le tableau ci-après récapitule les résultats des exemples précédents:

| Exemple n° | X | Y | rendement en éther XY |
|---|---|---|---|
| 1 | $4\text{-}NO_2$ | $2\text{-}C\begin{smallmatrix}\nearrow O\\ \searrow O\,\emptyset\end{smallmatrix}$ | 89% a) |
| 2 (comp.) | $4\text{-}NO_2$ | $2\text{-}C\begin{smallmatrix}\nearrow O\\ \searrow OCH_3\end{smallmatrix}$ | 36% b) |
| 3 | $4\text{-}NO_2$ | $3\text{-}NO_2$ | 75% a) |
| 4 | $4\text{-}NO_2$ | 4-CN | 82% a) |
| 5 (comp.) | $4\text{-}NO_2$ | 4-Cl | 10% b) |
| 6 | $4\text{-}NO_2$ | $4\text{-}SO_2\text{-}\ \emptyset$ | 90% a) |
| 7 (comp.) | $4\text{-}NO_2$ | $3,4\ \text{di-}C\begin{smallmatrix}\nearrow O\\ \searrow OCH_3\end{smallmatrix}$ | 14% b) |
| 8 | $4\text{-}NO_2$ | $2\ C\begin{smallmatrix}\nearrow O\\ \searrow O\,\emptyset\end{smallmatrix}$ | 71% a) |

a) rendement en produit recristallisé
b) rendement en produit isolé par chromatographie colonne de silice

Il met en évidence l'importance de la présence des groupes attracteurs sur les deux noyaux (comparer les exemples 1, 3, 4, 6 à l'exemple 5, dans lequel un des deux noyaux est substitué par un atome de chlore). Le tableau fait aussi apparaître l'importance du choix des groupes attracteurs, qui ne doivent être sensibles à l'action des bases (comparer l'exemple 1, dans lequel un des noyaux est substitué par un ester de phényle, à l'exemple 2, dans lequel le substituant est un ester de méthyle et à l'exemple 7, dans lequel le substituant est constitué de 2 groupements esters de méthyle.

*Exemple 9*

*Hydrogénation du 2-(4' -nitrophényl) benzoate de phényle*

Dans 50 $cm^3$ d'éthanol, on met en contact avec de l'hydrogène 9 g de 2-(4' -nitrophénoxy) benzoate de phényle préparé comme décrit dans l'exemple 1, en présence de 100 mg de palladium sur charbon à 5%. On obtient, après recristallisation dans 100 $cm^3$ de mélange méthanol-eau (50-50 en volumes), 7,52 g de 2(4' -aminophénoxy) benzoate de phényle (pf: 102°C, rendement 92%).

**Revendications**

1. Procédé de préparation d'un éther d'aryle dissymétrique répondant à la formule générale

X-Ar-O-Ar' -Y

dans laquelle Ar et Ar' représentent chacun un radical aromatique divalent comprenant un ou plusieurs cycles, X et Y représentent chacun un atome ou un groupement électro-attracteur différent l'un de l'autre par leur nature et/ou par leurs positions respectives sur les radicaux Ar et Ar', l'un au moins des substituants X et Y étant, sur le radical Ar ou Ar' qui le porte, en position ortho, para ou péri par rapport à la liaison avec l'atome d'oxygène, ledit procédé étant caractérisé en ce que l'on chauffe ensemble, en présence d'un catalyseur basique, des quantités sensiblement équimoléculaires de deux carbonates d'aryle répondant aux formules générales

X-Ar-O-CO-O-Ar-X
et Y-Ar' -O-CO-O-Ar' -Y

dans lesquelles Ar, Ar', X et Y sont définis comme précédemment; et en ce que l'on sépare l'éther d'aryle dissymétrique formé.

2. Procédé selon la revendication 1, dans lequel les radicaux Ar et Ar' sont des radicaux phénylène et l'un au moins des substituants X et Y est, sur le radical Ar ou Ar', en position ortho ou para par rapport à la liaison avec l'atome d'oxygène.

3. Procédé selon l'une des revendications 1 et 2, caractérisé en ce que X et Y sont choisis chacun parmi les groupements $-NO_2$, -CN, -COOAr'', et $-SO_2$-Ar'', où Ar'' représente un radical aromatique monovalent.

4. Procédé selon l'une des revendications 1 à 3, caractérisé en ce que X est un groupement $-NO_2$ et Y est choisi parmi les groupements $-NO_2$, -CN, -COOAr'', et $-SO_2$-Ar'', où Ar'' représente un radical aromatique monovalent.

5. Procédé selon l'une des revendications 1 à 4, caractérisé en ce que les deux carbonates de départ sont chauffés, à l'état fondu, à une température de 190 à 280°C.

6. Procédé selon l'une des revendications 1 à 4, caractérisé en ce que les deux carbonates de départ sont chauffés, en solution, à une température de 190 à 280°C.

7. Procédé selon l'une des revendications 1 à 6, caractérisé en ce que le catalyseur basique est choisi parmi les carbonates, les phénates, les acétates et les alcoolates des métaux alcalins.

8. Procédé selon l'une des revendications 1 à 7, caractérisé en ce que l'on utilise de 0,1 à 5 moles de catalyseur pour 100 moles de carbonates de départ.

9. Procédé selon l'une des revendications 1 à 8, caractérisé en ce que l'éther d'aryle est obtenu avec un rendement d'au moins 75%.

10. Procédé selon l'une des revendications 1 à 9, caractérisé en ce que l'on met en jeu deux carbonates d'aryle différents choisis chacun parmi
le bis-(4-nitrophényl)carbonate,
le bis-(3-nitrophényl)carbonate,
le bis-(4-cyanophényl)carbonate,
le bis-(2-phénoxy-carboxyphényl)carbonate, et
le bis-(4-phénylsulfonylphényl)carbonate.

11. Procédé selon la revendication 10, caractérisé en ce que l'un des deux carbonates d'aryle mis en jeu est le bis-(4-nitrophényl)carbonate et l'autre est choisi parmi:
le bis-(3-nitrophényl)carbonate,
le bis-(4-cyanophényl)carbonate,
le bis-(2-phénoxy-carboxyphényl)carbonate, et
le bis-(4-phénylsulfonylphényl)carbonate
et en ce que le catalyseur basique est du carbonate de potassium.

12. Procédé selon l'une des revendications 1 à 11, caractérisé en ce que l'on prépare un éther d'aryle dissymétrique de formule

X-Ar-O-Ar' -Y

dans laquelle au moins un des substituants X et Y est un groupement $-NO_2$ et en ce que ledit éther d'aryle est ensuite soumis à un traitement d'hydrogénation qui transforme les groupements $-NO_2$ en fonctions amines primaires.

**Patentansprüche**

1. Verfahren zur Herstellung eines unsymmetrischen Arylethers der allgemeinen Formel

X-Ar-O-Ar' -Y

worin bedeuten Ar und Ar' je einen zweiwertigen aromatischen Rest mit einem oder mehreren Ringen und X und Y je ein Atom oder eine Gruppe mit elektronenanziehenden Eigenschaften, die durch ihre Natur und/oder ihre entsprechenden Stellungen an den Resten Ar und Ar' voneinander verschieden sind, wobei sich mindestens einer der Substituenten X und Y an dem Rest Ar und Ar', der ihn trägt, in ortho-, para- oder peri-Stellung, bezogen auf die Bindung mit dem Sauerstoffatom, befindet, dadurch gekennzeichnet, dass man in Gegenwart eines basischen Katalysators praktisch equimolekulare Mengen von zwei Arylcarbonaten der allgemeinen Formeln

X-Ar-O-CO-O-Ar-X
und Y-Ar' -O-CO-O-Ar' -Y

worin Ar, Ar', X und Y die angegebene Bedeutung haben, zusammen erhitzt und den gebildeten unsymmetrischen Arylether abtrennt.

2. Verfahren nach Anspruch 1, dadurch gekennzeichnet, dass die Reste Ar und Ar' Phenylenreste sind und sich mindestens einer der Substituenten X und Y auf dem Rest Ar oder Ar' in ortho- oder para-Stellung, bezogen auf die Bindung mit dem Sauerstoffatom, befindet.

3. Verfahren nach einem der Ansprüche 1 und 2, dadurch gekennzeichnet, dass X und Y jeweils unter den Gruppen $-NO_2$, -CN, -COOAr" und $-SO_2$-Ar", worin Ar" einen einwertigen aromatischen Rest bedeutet, ausgewählt sind.

4. Verfahren nach einem der Ansprüche 1 bis 3, dadurch gekennzeichnet, dass X eine $-NO_2$ Gruppe bedeutet und Y unter den Gruppen $-NO_2$, -CN, -COOAr" und $-SO_2$-Ar", worin Ar" einen einwertigen aromatischen Rest bedeutet, ausgewählt ist.

5. Verfahren nach einem der Ansprüche 1 bis 4, dadurch gekennzeichnet, dass die beiden Ausgangscarbonate in aufgeschmolzenem Zustand bei einer Temperatur von 190 bis 280°C erhitzt werden.

6. Verfahren nach einem der Ansprüche 1 bis 4, dadurch gekennzeichnet, dass die beiden Ausgangscarbonate in Lösung bei einer Temperatur von 190 bis 280°C erhitzt werden.

7. Verfahren nach einem der Ansprüche 1 bis 6, dadurch gekennzeichnet, dass der basische Katalysator unter den Carbonaten, Phenolaten, Acetaten und Alkoholaten von Alkalimetallen ausgewählt ist.

8. Verfahren nach einem der Ansprüche 1 bis 7, dadurch gekennzeichnet, dass man 0,1 bis 5 mol Katalysator pro 100 mol Ausgangscarbonat einsetzt.

9. Verfahren nach einem der Ansprüche 1 bis 8, dadurch gekennzeichnet, dass der Arylether in einer Ausbeute von mindestens 75% anfällt.

10. Verfahren nach einem der Ansprüche 1 bis 9, dadurch gekennzeichnet, dass man zwei verschiedene Arylcarbonate einsetzt, die jeweils ausgewählt sind aus
Bis-(4-nitrophenyl)carbonat,
Bis-(3-nitrophenyl)carbonat,
Bis-(4-cyanophenyl)carbonat,
Bis-(2-phenoxycarboxyphenyl)carbonat und
Bis-(4-phenylsulfonylphenyl)carbonat.

11. Verfahren nach Anspruch 10, dadurch gekennzeichnet, dass man als eines der beiden Arylcarbonate Bis-(4-nitrophenyl)carbonat einsetzt und das andere auswählt aus:
Bis-(3-nitrophenyl)carbonat,
Bis-(4-cyanophenyl)carbonat,
Bis-(2-phenoxycarboxyphenyl)carbonat und
Bis-(4-phenylsulfonylphenyl)carbonat
und dass man als basischen Katalysator Kaliumcarbonat einsetzt.

12. Verfahren nach einem der Ansprüche 1 bis 11, dadurch gekennzeichnet, dass man einen unsymmetrischen Arylether der Formel

X-Ar-O-Ar' -Y

herstellt, worin mindestens einer der Substituenten X und Y eine $NO_2$-Gruppe ist und dass man den Arylether sodann einer Hydrierbehandlung unterwirft, die die $NO_2$-Gruppen in primäre Aminfunktionen überführt.

**Claims**

1. A process for the manufacture of an asymmetrical aryl ether of the general formula:

X-Ar-O-Ar' -Y

wherein Ar and Ar' are each a divalent aromatic radical having one or more rings, X and Y are each an electro-attracting atom or group differing from each other by their nature and/or by their respective positions on radicals Ar and Ar', at least one of the X and Y substituents being, on the Ar or Ar' radical, in ortho, para or peri position with respect to the oxygen atom bond, said process being characterized by heating together, in the presence of a basic catalyst, substantially equimolecular amounts of two aryl carbonates complying with the general formulae:

X-Ar-O-CO-O-Ar-X, and
Y-Ar' -O-CO-O-Ar' -Y

wherein Ar, Ar', X and Y are defined as above, and separating the asymmetrical aryl ether formed.

2. A process according to claim 1, wherein the Ar and Ar' radicals are phenylene radicals and at least one of the X and Y substituents on the Ar or Ar' radical is in ortho or para position with respect to the oxygen atom bond.

3. A process according to one of claims 1 and 2, characterized in that X and Y are selected from the $-NO_2$, -CN, -COOAr", $-SO_2$Ar" groups, wherein Ar" is a monovalent aromatic radical.

4. A process according to one of claims 1 to 3, characterized in that X is a $-NO_2$ group and Y is selected from the $-NO_2$, -CN, -COOAr", $-SO_2$Ar" groups, wherein Ar" is a monovalent aromatic radical.

5. A process according to one of claims 1 to 4, characterized in that the two starting carbonates are heated, in the molten state, at a temperature from 190 to 280°C.

6. A process according to one of claims 1 to 4, characterized in that the two starting carbonates are heated, as a solution, at a temperature from 190 to 280°C.

7. A process according to one of claims 1 to 6, characterized in that the basic catalyst is selected from the alkali metal carbonates, phenates, acetates and alcoholates.

8. A process according to one of claims 1 to 7, characterized in that the catalyst is used in an amount of 0.1 to 5 moles per 100 moles of the starting carbonates.

9. A process according to one of claims 1 to 8, characterized in that the aryl ether is obtained with a yield of at least 75%.

10. A process according to one of claims 1 to 9, characterized in that the two different aryl carbonates used are selected from:
bis-(4-nitrophenyl) carbonate,
bis-(3-nitrophenyl) carbonate,
bis-(4-cyanophenyl) carbonate,
bis-(2-phenoxy-carboxyphenyl) carbonate, and
bis-(4-phenylsulfonylphenyl) carbonate.

11. A process according to claim 10, characterized in that one of the two aryl carbonates used is bis-(4-nitrophenyl) carbonate, and the other is selected from:
bis-(3-nitrophenyl) carbonate,
bis-(4-cyanophenyl) carbonate,
bis-(2-phenoxy-carboxyphenyl) carbonate, and
bis-(4-phenylsulfonylphenyl) carbonate
and in that the basic catalyst is potassium carbonate.

12. A process according to one of claims 1 to 11, characterized by preparing an asymmetrical aryl ether of the formula

X-Ar-O-Ar'-Y

wherein at least one of the X and Y substituents is a $-NO_2$ group and subjecting said aryl ether to a subsequent hydrogenation step, converting the $-NO_2$ groups to primary amine groups.